# EUROPEAN PATENT SPECIFICATION

(11) **EP 3 882 600 B1**
(45) Date of publication and mention of the grant of the patent: **03.04.2024**
(21) Application number: 21159984.0
(22) Date of filing: 01.03.2021
(51) Int. Cl.: G01N 3/56, G01N 3/34, G01N 33/36

(54) **METHOD FOR SIMULATING THE AGEING OF A FABRIC**
VERFAHREN ZUR SIMULATION DER ALTERUNG EINES GEWEBES
PROCÉDÉ POUR SIMULER LE VIEILLISSEMENT D'UN TISSU

(30) Priority: 17.03.2020 IT 202000005650
(43) Date of publication of application: 22.09.2021
(73) Proprietor: Manteco S.p.A., 59013 Prato Montemurlo (IT)
(72) Inventor: MANTELLASSI, Marco, 59100 Prato (IT); MANTELLASSI, Matteo, 59100 Prato (IT); MANTELLASSI, Franco, 59100 Prato (IT)
(74) Representative: Firmati, Leonardo

(56) References cited:
- US-A1- 2015 299 905
- KADIR BILISIK ET AL: "Analysis and tensile-tear properties of abraded denim fabrics depending on pattern relations using statistical and artificial neural network models", FIBERS AND POLYMERS, THE KOREAN FIBER SOCIETY, HEIDELBERG, vol. 12, no. 3, 2 June 2011 (2011-06-02), pages 422-430, XP019911629, ISSN: 1875-0052, DOI: 10.1007/S12221-011-0422-8
- Unknown: "OPERATOR'S GUIDE AquAbrasion - Wet and Dry Abrasion Tester Featuring the Intuitive Touchscreen User Interface", , 1 January 2019 (2019-01-01), XP055746048, HALIFAX Retrieved from the Internet: URL:https://cdn2.hubspot.net/hubfs/3362727 /AquAbrasion%201819%20Operators%20Guide%20 -%20English.pdf [retrieved on 2020-11-02]
- Charlotte Davingoff: "AquAbrasion", , 24 April 2019 (2019-04-24), XP055746027, Retrieved from the Internet: URL:https://www.james-heal.co.uk/wp-conten t/uploads/2019/04/AquAbrasion-eBook-FINAL. pdf [retrieved on 2020-11-02]
- Charlotte Davingoff: "Document properties AquAbrasion eBook", , 2 November 2020 (2020-11-02), XP055746059, Retrieved from the Internet: URL:https://www.james-heal.co.uk/wp-conten t/uploads/2019/04/AquAbrasion-eBook-FINAL. pdf [retrieved on 2020-11-02]

## Description

This invention relates to a method for simulating the ageing of a fabric.

The need is increasingly felt to carry out quality checks on fabrics which have just come out from the production step, that is to say, before they are manufactured into clothing or furnishing items.

The above-mentioned control is performed in such a way as to obtain an assessment of the durability of a predetermined fabric and of how it can withstand so-called ageing, due both to the passing of time and its use.

There are no prior art testing and measurement procedures designed to provide an overall quality value of the resistance to ageing of a fabric but only a multiplicity of individual tests designed to quantify certain mechanical, physical or chemical features.

For example, in order to obtain a laceration resistance value of the fabric, the so-called Elmendorf pendulum lacerometer is known to be used.

The Elmendorf pendulum makes it possible to perform a controlled operation of tearing the fabric in question by sliding a pendulum calibrated by weight.

Depending on the resistance provided by the fabric to obstruct movement of the pendulum, the instrument detects a value identifying the resistance to laceration of the fabric in question.

Another known instrument in the textile sector is the Martindale type abrasion meter.

This abrasion meter comprises a plurality of sectors rotating on respective contact supports.

It is also possible to set a contact pressure in the above-mentioned abrasive meter in order to increase or decrease the abrasive effect.

The Martindale abrasion meter is used to derive quantitative values of the resistance to abrasion of the fabric in question.

By using the Martindale abrasion meter it is possible to also quantify the so-called pilling resistance of the fabric, that is to say, the quantification of the surface formation of bobbles, which when entangled form small lumps.

There are also other methods or instruments but are all designed to provide a value independent from the others, that is to say, a function of a single characteristic whether it is of a mechanical-physical type (abrasion, laceration, pilling...) or chemical type (colour migration, sweat fastness, washing fastness...).

KADIR BILISIK ET AL: "Analysis and tensile-tear properties of abraded denim fabrics depending on pattern relations using statistical and artificial neural network models",FIBERS AND POLYMERS, THE KOREAN FIBER SOCIETY, HEIDELBERG, vol. 12, no. 3, 2 June 2011, pages 422-430, ISSN: 1875-0052, DOI: 10.1007/ S12221-011-0422-8 discloses characterisation of mechanical properties of denim fabrics after abrasion load, wherein tensile and tear strengths of these fabrics are analysed using an Artificial Neural Network (ANN) and statistical model.

However, each single value obtained from the above-mentioned individual tests does not allow an overall qualitative value to be obtained which can be used as the simulation of the resistance to ageing of a fabric. The term "ageing" means the wear and the transformations which a fabric can withstand during its life cycle, that is to say, the durability of the fabric.

The aim of the invention is therefore to provide a method for simulating ageing of a predetermined fabric which is able to provide an overall and qualitative value of a single fabric before it is manufactured into a final product.

The above-mentioned aim therefore allows the manufacturer to select in an optimised manner the individual fabric according to the duration for which the manufactured garment is designed.

A further aim of the invention is to provide a method for simulating the ageing of a predetermined fabric which can be replicated and is repeatable in a systematic manner and which therefore allows a reliable and sure value of the resistance to ageing of a fabric to be obtained.

The above aims are achieved by a method for simulating the ageing of a predetermined fabric comprising the technical features described in one or more of the accompanying claims.

Further features and advantages of the invention are more apparent in the detailed description below, with reference to a preferred, non-limiting, embodiment of method for simulating the ageing of a predetermined fabric.

The method for simulating the ageing of a predetermined fabric according to the invention comprises the following steps, in the following precise order.

A first step comprises preparing two specimens of the predetermined fabric and subjecting them to an abrasion process by mutual rubbing.

The expression "mutual rubbing" means a rubbing between two specimens of the same predetermined fabric being tested.

This step allows simulation of the rubbing which occurs during normal use of the manufactured finished garment, for example at the user's armpit or in any case where two flaps of fabric are subjected to mutual rubbing. The method therefore comprises the step of subjecting a first of the two specimens already subjected to the mutual rubbing to a test by means of an Elmendorf lacerometer in the direction of the weft of the predetermined fabric, that is to say, positioning the specimen in the lacerometer in such a way that the tearing occurs in a direction perpendicular to the weft of the structure of the fabric under examination.

The method then comprises the step of subjecting the second of the two specimens to the same test by means of the Elmendorf pendulum abrasion meter, however this time in the direction of the warp of the predetermined fabric, that is to say, positioning the specimen in the lacerometer in such a way that tearing occurs in a direction perpendicular to the warp of the structure of the fabric under examination.

Advantageously, before carrying out the abrasion operation, the weft and warp directions are identified and marked on the reverse side of the two specimens. Once the results of the above-mentioned two tests have been obtained, the results are compared with a reference value, both of the weft and warp tear, relative to a previous Elmendorf test performed on the same predetermined fabric but not subjected to any abrasion process, that is to say "new".

Advantageously, the method according to the invention also comprises the steps of preparing a third specimen of the predetermined fabric and subjecting it to an abrasion process by rubbing against a reference abrasive fabric different from the fabric under examination.

Advantageously, the above-mentioned abrasion step takes place by applying a contact pressure of approximately 12 kPa and performing cycles of approximately 100,000 revolutions.

The method then comprises the step of subjecting the third specimen to a test using a Elmendorf pendulum type lacerometer and comparing the result of this test with a reference value relative to a previous test, again by means of an Elmendorf pendulum lacerometer, performed on a same predetermined fabric but not subjected to any abrasion process, that is to say "new".

Advantageously, the above-mentioned abrasion step is performed by a Martindale type abrasion meter.

If the predetermined fabric under examination is of a mainly protein or mainly cellulosic nature, the method according to the invention comprises further steps.

The expression "fabrics of a mainly protein nature" is used to mean all those fabrics comprising a greater percentage of natural fibres of animal origin, such as, for example: wool, cashmere, silk...

As mentioned, if, therefore, the predetermined fabric is of a mainly protein nature, the method also comprises the step of subjecting a fourth specimen of the fabric being examined to a bath in an aqueous solution with a pH of between 8 and 10, that is to say, an alkaline solution.

Advantageously, the fourth specimen is kept in a bath of the above-mentioned solution for 48 consecutive hours in such a way as to simulate the partial dissolving of the fibres of the fabric being examined.

Following the bath, the method comprises detecting some physical and dyeing features of the fourth specimen and, lastly, comparing them with the same physical and dyeing features detected on the same predetermined fabric not subjected to any process, that is to say "new".

Alternatively, if the predetermined fabric under examination is of a mainly cellulosic nature, the method according to the invention comprises further steps.

The expression "mainly cellulosic fabrics" means all those fabrics comprising a greater percentage of natural fibres of vegetable origin, such as, for example: linen, cotton, hemp...

As mentioned, if, therefore, the predetermined fabric is of a mainly cellulosic nature, the method also comprises the steps of subjecting a fourth specimen of the fabric being examined to a bath in an aqueous solution with a pH of between 3 and 5, that is to say, an acid solution. Advantageously, the fourth specimen is kept in a bath of the above-mentioned solution for 48 consecutive hours in such a way as to simulate the emptying of the fibres of the fabric being examined.

Following the bath, the method comprises detecting some physical and dyeing features of the fourth specimen and, lastly, comparing them with the same physical and dyeing features detected on the same predetermined fabric not subjected to any process, that is to say "new".

The term "physical features" means: tensile strength, laceration strength, seam creep strength, abrasion resistance and pilling resistance.

In detail:
- the tensile strength value is measured by means of a dynamometer applied to an instrument which pulls a specimen of fabric until it breaks,
- the laceration strength value is measured by means of a lacerometer, for example an Elmendorf pendulum,
- the seam creep strength is measured by applying a dynamometer to two previously stitched flaps of the fabric in question,
- the abrasion and pilling resistance values are measured by means of an abrasion meter, for example of the Martindale type.

The term "dyeing features" means: water fastness, sweat fastness, rubbing fastness, dry-cleaning fastness and washing fastness.

In detail:
- water fastness is measured by placing a specimen of fabric under examination which was wetted previously in contact with a multifibre fabric for a predetermined time, for example 4 hours, and then measuring the quantity of colour migrated from one to the other,
- sweat fastness is measured like water fastness, replacing the latter with a saline solution,
- rubbing fastness is measured by rubbing the specimen of fabric under examination with a portion of cotton fabric for a predetermined time and then measuring the quantity of colour migrated from one to the other,
- the washing fastness is measured by performing a series of normal washing cycles in water and solvent and
- dry-cleaning fastness is measured by inserting the specimen of fabric under examination inside a container with cotton and metal portions to which a quantity of perchloroethylene is added.

The assessment of colour degradation is tested according to the ISO standard grayscale (from 1 to 5) whilst assessment of the change in appearance of the fabric (from 1 to 5) is tested according to AATCC 124 and subsequent modifications.

The wearing of the garment manufactured with the fabric under examination is also simulated, subjecting it to 5 cold tumble dryer cycles. The wearing of the garment is thus simulated by the beating of the fabric against the walls of the tumble dryer and by the rubbing against itself.

At the end of the test the following features are verified: comparative assessment with the original of the colour degradation, comparative assessment with the original of the appearance of the fabric. The method according to the invention brings important advantages. An important advantage consists in the fact that the method according to the invention makes it possible to obtain an overall qualitative value of the resistance to ageing (from time and use) of a predetermined fabric. The above-mentioned value is obtained by means of the weighted sum of the results of the above-mentioned individual tests so as to provide a single qualitative value for each individual fabric.

A further advantage achieved by the method according to the invention is that it allows the end user of the predetermined fabric, for example the manufacturer of the item of clothing, to be able to choose the fabric in a completely knowledgeable manner according to the actual final use of the item of clothing.

## Claims

1. A method for simulating the ageing of a predetermined fabric comprising the following steps, in the precise order:
- preparing two specimens of said predetermined fabric,
- subjecting said specimens to an abrasion process by mutual rubbing,
- subjecting a first of said specimens to a test by means of an Elmendorf type lacerometer in the direction of the weft of said predetermined fabric,
- subjecting a second of said specimens to an Elmendorf test in the direction of the warp of said predetermined fabric,
- comparing the results of said Elmendorf tests with a reference value relative to an Elmendorf test performed on the same predetermined fabric not subjected to any abrasion process,
- preparing a third specimen of said fabric,
- subjecting said third specimen to an abrasion process by rubbing against a reference abrasive fabric,
- subjecting said third specimen to a laceration test using an Elmendorf pendulum abrasion meter,
- comparing the result of said Elmendorf test with a reference value relative to an Elmendorf test performed on the same predetermined fabric not subjected to any abrasion process.

2. The method according to claim 1, wherein said step of subjecting said third specimen to an abrasion process by rubbing against a reference abrasive fabric occurs by applying a contact pressure of approximately 12 kPa and performing cycles of approximately 100,000 revolutions.

3. The method according to claim 1 comprising also, if said determined fabric is mainly protein in nature, the steps of:
- subjecting a fourth specimen of said predetermined fabric to a bath in an aqueous solution with a pH of between 8 and 10,
- detecting physical and dyeing features of said fourth specimen and
- comparing said physical and dyeing features detected of said fourth specimen with the physical and dyeing features detected on the same predetermined fabric not subjected to any process.

4. The method according to claim 1, also comprising, if said predetermined fabric is of a mainly cellulosic nature, the steps of:
- subjecting a fourth specimen of said predetermined fabric to a bath in an aqueous solution with a pH of between 3 and 5,
- detecting physical and dyeing features of said fourth specimen and
- comparing said physical and dyeing features detected of said fourth specimen with the physical and dyeing features detected on the same predetermined fabric not subjected to any process.

5. The method according to claim 2 or 3, wherein said physical features detected are: tensile strength, laceration strength, seam creep strength, abrasion resistance and pilling strength.

6. The method according to the preceding claim, wherein said tensile strength is measured by means of a dynamometer applied to an instrument which pulls one of said specimens of fabric until it breaks.

7. The method according to claim 4, wherein said seam creep strength is measured by means of a dynamometer applied to two previously stitched flaps of one of said specimens.

8. The method according to claim 2 or 3, wherein said dyeing features detected are: water fastness, sweat fastness, rubbing fastness, dry-cleaning fastness and washing fastness.

9. The method according to the preceding claim, wherein said water fastness is measured by placing one of the specimens of the fabric previously wetted into contact with a multifibre fabric for a predetermined time and then measuring the quantity of colour migrated from said specimen to said multifibre fabric.

10. The method according to claim 7, wherein said rubbing fastness is measured by rubbing for a predetermined length of time one of said predetermined specimens of fabric with a portion of cotton fabric and then measuring the quantity of colour migrated from said specimen to said cotton portion.

11. The method according to any one of the preceding claims, wherein said abrasion processes by rubbing are carried out by means of an abrasion meter of the Martindale type.

## Patentansprüche

1. Verfahren zur Simulation der Alterung eines vorbestimmten Gewebes, umfassend in der genauen Reihenfolge die folgenden Schritte:
- Vorbereiten von zwei Proben des vorbestimmten Gewebes;
- Unterziehen dieser Proben einem Abriebprozess durch gegenseitiges Reiben;
- Unterziehen einer ersten dieser Proben einem Test mittels eines ballistischen Pendels nach Elmendorf in der Schussrichtung des vorbestimmten Gewebes;
- Unterziehen einer zweiten dieser Proben einem Elmendorf-Test in der Kettrichtung des vorbestimmten Gewebes;
- Vergleichen der Ergebnisse der Elmendorf-Tests mit einem Referenzwert, der sich auf einen Elmendorf-Test bezieht, der mit demselben vorbestimmten Gewebe durchgeführt wurde, das keinem Abriebprozess unterzogen wurde;
- Vorbereiten einer dritten Probe des Gewebes;
- Unterziehen der dritten Probe einem Abriebprozess, indem diese gegen ein scheuerndes Referenzgewebe gerieben wird;
- Unterziehen der dritten Probe einem Test zur Bestimmung der Weiterreißfestigkeit mittels eines ballistischen Pendels nach Elmendorf;
- Vergleichen des Ergebnisses des Elmendorf-Tests mit einem Referenzwert, der sich auf einen Elmendorf-Test bezieht, der mit demselben vorbestimmten Gewebe durchgeführt wurde, das keinem Abriebprozess unterzogen wurde.

2. Verfahren nach Anspruch 1, wobei der Schritt zum Unterziehen der dritten Probe einem Abriebprozess, indem diese gegen ein scheuerndes Referenzgewebe gerieben wird, erfolgt, indem ein Kontaktdruck von ungefähr 12 kPa angewandt wird und Zyklen von ungefähr 100.000 Umdrehungen durchgeführt werden.

3. Verfahren nach Anspruch 1, umfassend auch die folgenden Schritte, wenn es sich beim bestimmten Gewebe um eins handelt, das vorwiegend aus Proteinen besteht:
- Unterziehen einer vierten Probe des vorbestimmten Gewebes einem Bad in einer wässrigen Lösung mit einem pH-Wert zwischen 8 und 10,
- Erfassen von physikalischen und Färbungsmerkmalen der vierten Probe, und
- Vergleichen dieser erfassten physikalischen und Färbungsmerkmale der vierten Probe mit den physikalischen und Färbungsmerkmalen, die am selben vorbestimmten Gewebe erfasst wurden, das keinem Prozess unterzogen wurde.

4. Verfahren nach Anspruch 1, umfassend auch die folgenden Schritte, wenn es sich beim vorbestimmten Gewebe um eins handelt, das vorwiegend aus Zellulose besteht:
- Unterziehen einer vierten Probe des vorbestimmten Gewebes einem Bad in einer wässrigen Lösung mit einem pH-Wert zwischen 3 und 5,
- Erfassen von physikalischen und Färbungsmerkmalen der vierten Probe, und
- Vergleichen dieser erfassten physikalischen und Färbungsmerkmale der vierten Probe mit den physikalischen und Färbungsmerkmalen, die am selben vorbestimmten Gewebe erfasst wurden, das keinem Prozess unterzogen wurde.

5. Verfahren nach Anspruch 2 oder 3, wobei es sich bei den erfassten physikalischen Merkmalen um folgende handelt: Reißfestigkeit, Weiterreißfestigkeit, Nahtschiebefestigkeit, Abriebfestigkeit und Pillingfestigkeit.

6. Verfahren nach dem vorhergehenden Anspruch, wobei die Reißfestigkeit mittels eines Festigkeitsprüfers bestimmt wird, der an einem Gerät angebracht ist, das eine der Gewebeproben zieht, bis sie reißt.

7. Verfahren nach Anspruch 4, wobei die Nahtschiebefestigkeit mittels eines Festigkeitsprüfers bestimmt wird, der an zwei zuvor genähten Laschen von einer der Proben angebracht wird.

8. Verfahren nach Anspruch 2 oder 3, wobei es sich bei den erfassten Färbungsmerkmalen um folgende handelt: Wasserechtheit, Schweißechtheit, Reibechtheit, Farbechtheit gegen Trockenreinigung und Farbechtheit gegen Waschen.

9. Verfahren nach dem vorhergehenden Anspruch, wobei die Wasserechtheit bestimmt wird, indem eine der Proben des Gewebes, die zuvor benetzt wurde, für einen vorbestimmten Zeitraum in Kontakt mit einem mehrfaserigen Gewebe gebracht und dann die Menge der Farbe bestimmt wird, die von der Probe auf das mehrfaserige Gewebe übergegangen ist.

10. Verfahren nach Anspruch 7, wobei die Reibechtheit bestimmt wird, indem eine der vorbestimmten Gewebeproben eine vorbestimmte Zeitdauer mit einem Baumwollgewebeabschnitt gerieben wird und dann die Menge der Farbe bestimmt wird, die von der Probe auf den Baumwollabschnitt übergegangen ist.

11. Verfahren nach einem der vorhergehenden Ansprüche, wobei die Abriebprozesse durch Reiben mittels eines Martindale-Prüfgeräts durchgeführt werden.

## Revendications

1. Procédé pour simuler le vieillissement d'un tissu prédéterminé, comprenant les étapes suivantes, dans l'ordre précis :
- préparer deux échantillons dudit tissu prédéterminé,
- soumettre lesdits échantillons à un processus d'abrasion par frottement mutuel,
- soumettre un premier desdits échantillons à un test au moyen d'un testeur de déchirure de type Elmendorf dans le sens de la trame dudit tissu prédéterminé,
- soumettre un deuxième desdits échantillons à un test Elmendorf dans le sens de la chaîne dudit tissu prédéterminé,
- comparer les résultats desdits tests Elmendorf à une valeur de référence relative à un test Elmendorf effectué sur le même tissu prédéterminé non soumis à un quelconque processus d'abrasion,
- préparer un troisième échantillon dudit tissu,
- soumettre ledit troisième échantillon à un processus d'abrasion en le frottant contre un tissu abrasif de référence,
- soumettre ledit troisième échantillon à un test de déchirure à l'aide d'un abrasimètre à pendule tombant d'Elmendorf,
- comparer le résultat dudit test Elmendorf à une valeur de référence relative à un test Elmendorf effectué sur le même tissu prédéterminé non soumis à un quelconque processus d'abrasion,

2. Procédé selon la revendication 1, dans lequel ladite étape consistant à soumettre ledit troisième échantillon à un processus d'abrasion par frottement contre un tissu abrasif de référence s'effectue en appliquant une pression de contact d'environ 12 kPa et en effectuant des cycles d'environ 100 000 tours.

3. Procédé selon la revendication 1, comprenant également, si ledit tissu déterminé est principalement de nature protéique, les étapes de :
- soumettre un quatrième échantillon dudit tissu prédéterminé à un bain dans une solution aqueuse dont le pH est compris entre 8 et 10,
- détecter les caractéristiques physiques et tinctoriales dudit quatrième échantillon, et
- comparer lesdites caractéristiques physiques et tinctoriales détectées dudit quatrième échantillon avec les caractéristiques physiques et tinctoriales détectées sur le même tissu prédéterminé n'ayant subi aucun traitement.

4. Procédé selon la revendication 1, comprenant également, si ledit tissu prédéterminé est de nature principalement cellulosique, les étapes de :
- soumettre un quatrième échantillon dudit tissu prédéterminé à un bain dans une solution aqueuse dont le pH est compris entre 3 et 5,
- détecter les caractéristiques physiques et tinctoriales dudit quatrième échantillon, et
- comparer lesdites caractéristiques physiques et tinctoriales détectées dudit quatrième échantillon avec les caractéristiques physiques et tinctoriales détectées sur le même tissu prédéterminé n'ayant subi aucun traitement.

5. Procédé selon la revendication 2 ou 3, dans lequel lesdites caractéristiques physiques détectées sont : la résistance à la traction, la résistance à la déchirure, la résistance au fluage des coutures, la résistance à l'abrasion et la résistance au boulochage.

6. Procédé selon la revendication précédente, dans lequel ladite résistance à la traction est mesurée au moyen d'un dynamomètre appliqué à un instrument qui tire l'un desdits échantillons de tissu jusqu'à ce qu'il se casse.

7. Procédé selon la revendication 4, dans lequel ladite résistance au fluage des coutures est mesurée au moyen d'un dynamomètre appliqué à deux rabats préalablement cousus de l'un desdits échantillons.

8. Procédé selon la revendication 2 ou 3, dans lequel lesdites caractéristiques tinctoriales détectées sont : la solidité à l'eau, la solidité à la sueur, la solidité au frottement, la solidité au nettoyage à sec et la solidité au lavage.

9. Procédé selon la revendication précédente, dans lequel ladite solidité à l'eau est mesurée en plaçant l'un des échantillons du tissu préalablement mouillé en contact avec un tissu multifibre pendant une durée prédéterminée et en mesurant ensuite la quantité de couleur migrée dudit échantillon vers ledit tissu multifibre.

10. Procédé selon la revendication 7, dans lequel ladite solidité au frottement est mesurée en frottant pendant une durée prédéterminée l'un desdits échantillons de tissu prédéterminés avec une partie de tissu en coton, puis en mesurant la quantité de couleur migrée dudit échantillon vers ladite partie de coton.

11. Procédé selon l'une quelconque des revendications précédentes, dans lequel lesdits processus d'abrasion par frottement sont effectués au moyen d'un abrasimètre du type Martindale.
